# EUROPEAN PATENT APPLICATION

(11) **EP 4 321 505 A1**
(43) Date of publication of application: **14.02.2024**
(21) Application number: 22925230.9
(22) Date of filing: 17.06.2022
(51) Int. Cl.: C07D 215/10, C07D 209/44, C07D 223/04, C07D 211/14, C07D 213/20, C07C 211/63, C07C 211/64

(54) **IONIC COMPOUND AND USE THEREOF, PEROVSKITE PRECURSOR SOLUTION, PEROVSKITE MATERIAL, SOLAR CELL, AND ELECTRICAL DEVICE**

(71) Applicant: Contemporary Amperex Technology Co., Ltd., Ningde, Fujian (CN)
(72) Inventor: LI, Hanfang, Ningde, Fujian 352100 (CN); SU, Shuojian, Ningde, Fujian 352100 (CN); LIANG, Weifeng, Ningde, Fujian 352100 (CN); CHEN, Changsong, Ningde, Fujian 352100 (CN); LIN, Xiangling, Ningde, Fujian 352100 (CN); LIU, Zhaohui, Ningde, Fujian 352100 (CN); CHEN, Guodong, Ningde, Fujian 352100 (CN); GUO, Yongsheng, Ningde, Fujian 352100 (CN); GUO, Yongsheng, Ningde, Fujian 352100 (CN)
(74) Representative: Jacob, Reuben Ellis
(86) International application number: PCT/CN2022/099567
(87) International publication number: WO 2023/240629

(57) **Abstract**

Provided are an ionic compound and an application thereof, a perovskite precursor solution, a perovskite material, a solar material, a solar cell, and an electric apparatus. The ionic compound has a structure represented by any one of formulas (1) to (3). The ionic compound can improve the stability of perovskite materials and thus improve the efficiency and stability of solar cells.

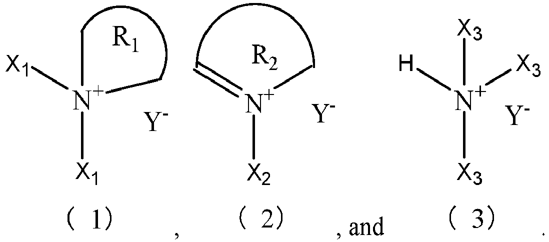

## Description

### TECHNICAL FIELD

This application relates to the field of batteries, and specifically, to an ionic compound and an application thereof, a perovskite precursor solution, a perovskite material, a solar cell, and an electric apparatus.

### BACKGROUND

With the rapid development of photovoltaic technologies and perovskite materials, perovskite solar cells have emerged on the basis of photoelectrochemistry and perovskite materials. In perovskite solar cells, perovskite materials are used to prepare the light-absorbing layer. Therefore, compared with solar cells whose light-absorbing layer is made of other materials, perovskite solar cells have many advantages. First, perovskite materials have a regular structure, absorb a quite large portion of the visible spectrum, and well match the energy level of titanium dioxide and other semiconductors, so that visible light can be exploited to the maximum extent and resulting perovskite solar cells have high efficiency. Second, perovskite solar cells can have a solid hole transport layer, and therefore, are simple and fast to prepare and cost-effective, and have much less packaging difficulty than other solar cells with a liquid hole layer. In addition, perovskite solar cells have a wide range of raw materials, and can be used to prepare large-area flexible batteries and transparent batteries.

The perovskite light-absorbing layer is generally formed through the solution method. During film formation, arrangement of structural units of a perovskite material inevitably experiences an error, resulting in crystal defects in the resulting light-absorbing layer. This directly affects processes such as exciton generation and separation, charge carrier diffusion, and charge transport in perovskite solar cells and in turn reduces the efficiency and stability of perovskite solar cells.

Therefore, the prior art still needs to be improved.

### SUMMARY

In view of the above problems, this application provides an ionic compound and an application thereof, a perovskite precursor solution, a perovskite material, a solar cell, and an electric apparatus. The ionic compound can improve the stability of perovskite materials and thus improve the efficiency and stability of solar cells.

According to a first aspect, this application provides an ionic compound. The ionic compound has a structure represented by any one of formulas (1) to (3): 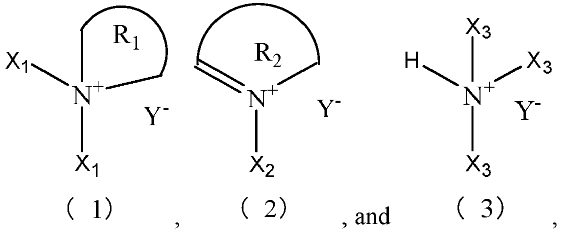
where X₁ is independently selected at each occurrence from any one of H or linear alkane group having 1 to 50 carbon atoms; R₁ and N are bonded to each other to form an alicyclic heterocyclic ring unsubstituted or substituted by linear alkane, the alicyclic heterocyclic ring having 7 to 50 ring atoms and containing only one N atom; and at least one X₁ in formula (1) is not H;
X₂ is selected from linear alkane group having 1 to 50 carbon atoms, and R₂ and N are bonded to each other to form an aromatic heterocyclic ring unsubstituted or substituted by linear alkane, the aromatic heterocyclic ring having 7 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

The ionic compound of this application contains one N atom having lone pair electrons, forms a specific ring structure, and is organically bonded to linear alkane. The lone pair electrons of the N atom can be coordinated with vacancy-containing lead ions in a perovskite material, thereby reducing non-radiative recombination. The linear alkane can be bonded to free H formed in the perovskite material, avoiding the formation of acidoids such as hydroiodic acid by free H and free iodine ions. In addition, the organic bond of the aromatic heterocyclic ring or alicyclic ring and the linear alkane makes the ionic compound have excellent hydrophobicity, which can effectively prevent water in the environment from entering the inside of the perovskite material through a grain boundary or another defective site while such entry leads to decomposition of the perovskite material, improve the stability of perovskite layers, and reduce defects, thereby improving the efficiency and stability of solar cells.

R₁ and N are bonded to each other to form an alicyclic heterocyclic ring unsubstituted or substituted by linear alkane, the alicyclic heterocyclic ring having 7 to 20 ring atoms and containing only one N atom; and/or
R₂ and N are bonded to each other to form a dense aromatic heterocyclic ring unsubstituted or substituted by linear alkane, the dense aromatic heterocyclic ring having 8 to 20 ring atoms and containing only one N atom.

In some embodiments, X₃ is independently selected at each occurrence from any one of H, aromatic ring group substituted by straight-chain alkane and having 6 to 20 ring atoms, alicyclic alkyl group substituted by straight-chain alkane and having 6 to 20 ring atoms, straight-chain alkane group substituted by aromatic ring and having 1 to 20 carbon atoms, or straight-chain alkane group substituted by alicyclic alkyl and having 1 to 20 carbon atoms, and at least one X₃ in formula (3) is not H.

In some embodiments, the structure of formula (3) is shown as follows: 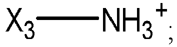 where X₃ is selected from any one of aromatic ring group substituted by straight-chain alkane and having 6 to 20 ring atoms or alicyclic alkyl group substituted by straight-chain alkane and having 6 to 20 ring atoms.

In some embodiments, the ionic compound has a structure represented by formulas (B) to (E): 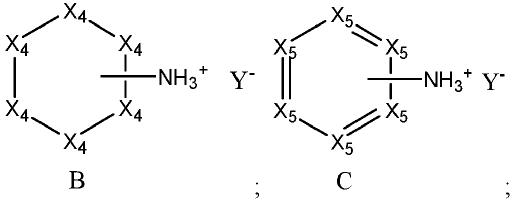 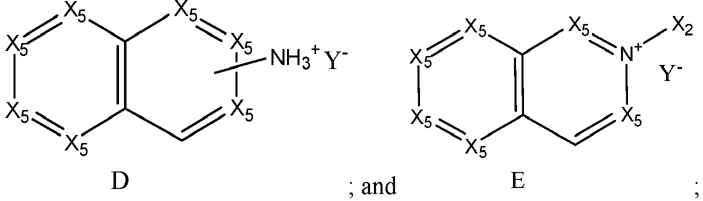
where X₄ is independently selected at each occurrence from CR₃R₄, and X₅ is independently selected at each occurrence from CR₅; and
R₃ to R₅ each are independently selected at each occurrence from any one of H or linear alkyl group having 1 to 30 carbon atoms; at least one R₃ or at least one R₄ in formula (B) is selected from linear alkyl group having 1 to 30 carbon atoms; and at least one R₅ in formula (C) and formula (D) is selected from linear alkyl group having 1 to 30 carbon atoms.

In some embodiments, R₃ to R₅ each are independently selected at each occurrence from H or straight-chain alkyl group having 1 to 15 carbon atoms; at least R₃ or at least one R₄ in formula (B) is selected from straight-chain alkyl group having 1 to 15 carbon atoms; and at least one R₅ in formula (C) and formula (E) is selected from straight-chain alkyl group having 1 to 15 carbon atoms.

In some embodiments, Y⁻ is selected from fluoroborate containing ion or fluorophosphate containing ion.

According to a second aspect, this application provides an application of the ionic compound according to the first aspect in an ionic liquid.

According to a third aspect, this application provides an application of the ionic compound represented by any one of formulas (3) to (5) in perovskite material preparation: 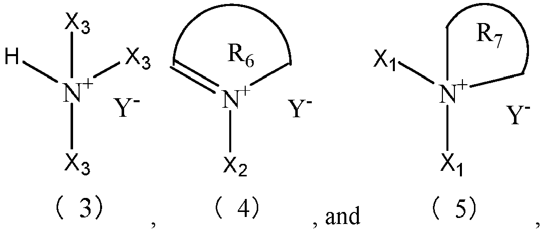
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

According to a fourth aspect, this application provides a perovskite precursor solution. Compositions of the perovskite precursor solution include a perovskite precursor and at least one of ionic compounds represented by (3) to (5): 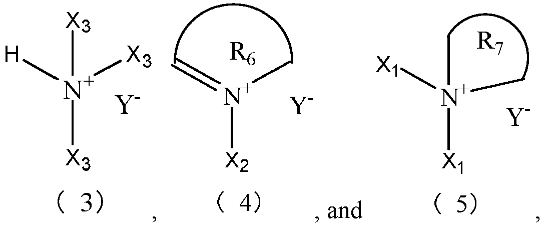
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

According to a fifth aspect, this application provides a perovskite material. Compositions of the perovskite material include a perovskite compound and at least one of ionic compounds represented by formulas (3) to (5): 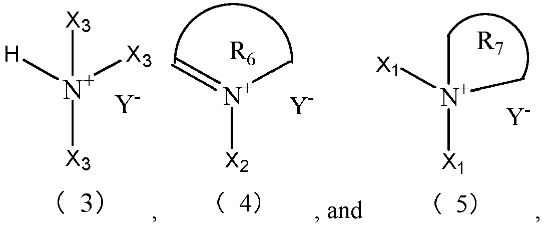
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

According to a sixth aspect, this application provides a preparation method for the perovskite material according to the fifth aspect. The method includes the following steps:
mixing a perovskite precursor, the ionic compound, and a solvent to obtain a perovskite precursor solution; and
annealing the perovskite precursor solution to obtain the perovskite material.

According to a seventh aspect, this application provides a solar cell. The solar cell includes the perovskite material according to the fifth aspect.

According to an eighth aspect, this application provides an electric apparatus. The electric apparatus includes the solar cell according to the seventh aspect.

The foregoing description is merely an overview of the technical solution of this application. For a better understanding of the technical means in this application such that they can be implemented according to the content of the specification, and to make the above and other objectives, features and advantages of this application more obvious and easier to understand, the following describes specific embodiments of this application.

### BRIEF DESCRIPTION OF DRAWINGS

Persons of ordinary skill in the art can clearly understand various other advantages and benefits by reading the detailed description of the preferred embodiments below. The accompanying drawings are merely intended to illustrate the preferred embodiments and are not intended to limit this application. In addition, in all the accompanying drawings, like parts are denoted by like reference signs. In the accompanying drawings:
FIG. 1 is a schematic diagram of a solar cell according to an embodiment of this application; and
FIG. 2 is a schematic diagram of an electric apparatus using a solar cell as a power source according to an embodiment of this application.

Description of reference signs:
10. solar cell; 11. first electrode; 12. electron transport layer; 13. perovskite layer; 14. hole transport layer; 15. second electrode; and 20. electric apparatus.

### DESCRIPTION OF EMBODIMENTS

The following describes in detail the embodiments of technical solutions of this application with reference to the accompanying drawings. The following embodiments are merely intended for a clearer description of the technical solutions of this application and therefore are used as just examples which do not constitute any limitations on the protection scope of this application.

Unless otherwise defined, all technical and scientific terms used herein shall have the same meanings as commonly understood by those skilled in the art to which this application relates. The terms used herein are intended to merely describe the specific embodiments rather than to limit this application. The terms "include", "include", and "have" and any other variations thereof in the specification, claims and brief description of drawings of this application are intended to cover non-exclusive inclusions.

In the description of the embodiments of this application, the terms "first", "second" and the like are merely intended to distinguish between different objects, and shall not be understood as any indication or implication of relative importance or any implicit indication of the number, sequence or primary-secondary relationship of the technical features indicated. In the description of the embodiments of this application, "a plurality of" means at least two unless otherwise specifically stated.

In this specification, reference to "embodiment" means that specific features, structures or characteristics described with reference to the embodiment may be incorporated in at least one embodiment of this application. The word "embodiment" appearing in various places in the specification does not necessarily refer to the same embodiment or an independent or alternative embodiment that is exclusive of other embodiments. It is explicitly or implicitly understood by persons skilled in the art that the embodiments described herein may be combined with other embodiments.

In the description of the embodiments of this application, the term "and/or" is only an associative relationship for describing associated objects, indicating that three relationships may be present. For example, A and/or B may indicate the following three cases: presence of only A, presence of both A and B, and presence of only B. In addition, the character "/" in this specification generally indicates an "or" relationship between contextually associated objects.

In the description of the embodiments of this application, the term "a plurality of" means more than two (inclusive). Similarly, "a plurality of groups" means more than two (inclusive) groups, and "a plurality of pieces" means more than two (inclusive) pieces.

In the description of the embodiments of this application, the orientations or positional relationships indicated by the technical terms "center", "longitudinal", "transverse", "length", "width", "thickness", "upper", "lower", "front", "rear", "left", "right", "vertical", "horizontal", "top", "bottom", "inside", "outside", "clockwise", "counterclockwise", "axial", " radial", "circumferential", and the like are based on the orientations or positional relationships as shown in the accompanying drawings. These terms are merely for ease and brevity of description of the embodiments of this application rather than indicating or implying that the means or components mentioned must have specific orientations or must be constructed or manipulated according to specific orientations, and therefore shall not be construed as any limitations on embodiments of this application.

In the description of the embodiments of this application, unless otherwise specified and defined explicitly, the terms "mount", "connect", "join", and "fasten" should be understood in their general senses. For example, they may refer to a fixed connection, a detachable connection, or an integral connection, may refer to a mechanical connection or electrical connection, and may refer to a direct connection, an indirect connection via an intermediate medium, or an interaction between two elements. Persons of ordinary skill in the art can understand specific meanings of these terms in the embodiments of this application as appropriate to specific situations.

In the present invention, the term "linear alkane group" refers to a group formed after linear alkane loses one hydrogen. For example, methane loses one hydrogen to form a methyl group. The term "linear alkane" refers to alkane in which carbon atoms are all connected by carbon-carbon single bonds, with no ring formed and remaining valence bonds all bonded to hydrogen, including straight-chain alkane and branched alkane.

In the present invention, "linear alkane group" may have 1 to 50 carbon atoms, including 1, 2, 3, 4, 5, 6, 7, 8, 9, 10, 20, 30, 40, and 50, and means a group formed after linear alkane containing 1 to 50 carbon atoms (that is, C1 to C50 linear alkane) loses one hydrogen. Non-limiting examples of "C1 to C50 linear alkane" include methane, ethane, n-propane, isopropane, n-butane, isobutane, 2-ethylbutane, 3,3-dimethylbutane, n-pentane, isopentane, neopentane, 1-methylpentane, 3-methylpentane, 2-ethylpentane, 4-methyl-2-pentane, n-hexane, 1-methylhexane, 2-ethylhexane, 2-butylhexane, n-heptane, 1-methylheptane, 2,2-dimethylheptane, 2-ethylheptane, n-octane, n-nonane, and n-decane. In other words, non-limiting examples of "C1 to C50 linear alkane group" include groups formed after the foregoing linear alkane loses one hydrogen.

In the present invention, the "number of ring atoms" indicates the number of atoms bonded into a ring. When the ring is replaced by a substituent, atoms included in the substituent are not included in the ring-forming atoms. The same applies to the "number of ring atoms" as described below, unless otherwise specified. For example, a benzene ring has 6 ring atoms, a naphthalene ring has 10 ring atoms, and thiophene has 5 ring atoms.

In the present invention, when a group is selected from "aromatic ring group substituted by linear alkane and having 6 to 50 ring atoms", a site where the group is bonded to another part of a molecule is located at the aromatic ring group. Similarly, when a group is selected from "alicyclic alkyl group substituted by linear alkane and having 6 to 50 ring atoms", a site where the group is bonded to another part of a molecule is located at the alicyclic alkyl group. When a group is selected from "linear alkane group substituted by aromatic ring and having 1 to 50 carbon atoms" and "linear alkane group substituted by alicyclic alkyl and having 1 to 50 carbon atoms", a site where the group is bonded to another part of a molecule is located at the linear alkane group. In other cases, if a bond site is not specified in the group, it indicates that any bond site in the group is selected as a bond site.

In the present invention, a single bond to a substituent runs through a corresponding ring. This indicates that the substituent can be bonded to any site of the ring. For example, in 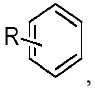 R is bonded to any one of substitutable sites of the benzene ring.

In the present invention, "substituted or unsubstituted" indicates that the defined group may or may not be substituted.

As described in the background, during film formation, arrangement of structural units of a perovskite material inevitably experiences an error, resulting in crystal defects in the resulting light-absorbing layer, including bulk defects and interface defects, and in turn reducing the efficiency and stability of perovskite solar cells.

In conventional technologies, interfacial passivation is often used to improve the efficiency and stability of battery devices. Usually, a functional passivation layer is added on the surface of a perovskite layer or a functional inhibitor is directly added to a perovskite material to inhibit the generation of defects in the perovskite material. However, technical staff of this application have found in actual production and research and development that in conventional technologies, an ionic compound containing a five-membered heterocyclic ring is typically used to prepare a functional passivation film or directly added to a perovskite material, but a solar cell prepared sees limited improvement in photoconversion efficiency and stability. After a lot of creative experiments, the technical staff of this application have found that the structure of a ring in an ionic compound and the hetero atom type and number of the ring have great influence on the suppression and repair of defects in a perovskite material.

Therefore, through extensive creative experiments, the technical staff of this application have obtained an ionic compound that can further improve the stability of a perovskite material in this application, and thus improve the efficiency and stability of solar cells.

An embodiment of the present invention provides an ionic compound. The ionic compound has a structure represented by any one of formulas (1) to (3): 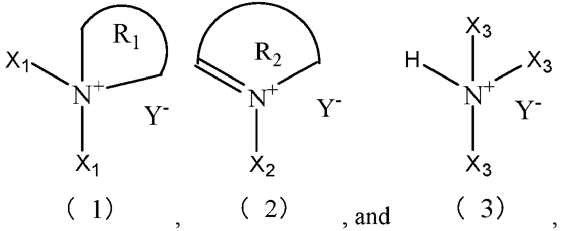
where X₁ is independently selected at each occurrence from any one of H or linear alkane group having 1 to 50 carbon atoms; R₁ and N are bonded to each other to form an alicyclic heterocyclic ring unsubstituted or substituted by linear alkane, the alicyclic heterocyclic ring having 7 to 50 ring atoms and containing only one N atom; and at least one X₁ in formula (1) is not H;
X₂ is selected from linear alkane group having 1 to 50 carbon atoms, and R₂ and N are bonded to each other to form an aromatic heterocyclic ring unsubstituted or substituted by linear alkane, the aromatic heterocyclic ring having 7 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

The ionic compound contains only one N atom, forms a specific ring structure, and is organically bonded to linear alkane. The lone pair electrons of the N atom can be coordinated with vacancy-containing lead ions in a perovskite material, thereby reducing non-radiative recombination. The linear alkane can be bonded to free H formed in the perovskite material, avoiding the formation of acidoids such as hydroiodic acid by free H and free iodine ions. In addition, the organic bond of the aromatic heterocyclic ring or alicyclic ring and the linear alkane makes the ionic compound have excellent hydrophobicity, which can effectively prevent water in the environment from entering the inside of the perovskite material through a grain boundary or another defective site while such entry leads to decomposition of the perovskite material, improve the stability of perovskite layers, and reduce defects, thereby improving the efficiency and stability of solar cells.

In some embodiments, R₁ and N are bonded to each other to form an alicyclic heterocyclic ring unsubstituted or substituted by linear alkane, the alicyclic heterocyclic ring having 7 to 20 ring atoms and containing only one N atom.

It should be noted that the above range is considered continuous and includes a minimum value and a maximum value of the range, as well as each value between such minimum value and maximum value, including but not limited to the point values in this embodiment. For example, the foregoing "7 to 20 ring atoms" may be 7, 8, 9, 10, 11, 12, 13, 14, 15, 16, 17, 18, 18, or 20 ring atoms.

In some embodiments, R₁ and N are bonded to each other to form an alicyclic heterocyclic ring unsubstituted or substituted by C1-C20 linear alkane, the alicyclic heterocyclic ring having 7 to 10 ring atoms and containing only one N atom.

In some embodiments, R₁ and N are bonded to each other to form an alicyclic heterocyclic ring unsubstituted or substituted by C1-C10 straight-chain alkane, the alicyclic heterocyclic ring having 7 to 10 ring atoms and containing only one N atom.

In some embodiments, R₂ and N are bonded to each other to form a dense aromatic heterocyclic ring unsubstituted or substituted by linear alkane, the dense aromatic heterocyclic ring having 8 to 20 ring atoms and containing only one N atom.

In some embodiments, R₂ and N are bonded to each other to form a dense aromatic heterocyclic ring unsubstituted or substituted by straight-chain alkane, the dense aromatic heterocyclic ring having 8 to 20 ring atoms and containing only one N atom.

In some embodiments, R₂ and N are bonded to each other to form a dense aromatic heterocyclic ring unsubstituted or substituted by C1-C20 straight-chain alkane, the dense aromatic heterocyclic ring having 8 to 15 ring atoms and containing only one N atom.

In some embodiments, R₂ and N are bonded to each other to form a dense aromatic heterocyclic ring unsubstituted or substituted by C1-C10 straight-chain alkane, the dense aromatic heterocyclic ring having 10 to 15 ring atoms and containing only one N atom.

In some embodiments, X₃ is independently selected at each occurrence from any one of H, aromatic ring group substituted by straight-chain alkane and having 6 to 20 ring atoms, alicyclic alkyl group substituted by straight-chain alkane and having 6 to 20 ring atoms, straight-chain alkane group substituted by aromatic ring and having 1 to 20 carbon atoms, or straight-chain alkane group substituted by alicyclic alkyl and having 1 to 20 carbon atoms, and at least one X₃ in formula (3) is not H.

In some embodiments, X₃ is independently selected at each occurrence from any one of H, aromatic ring group substituted by straight-chain alkane and having 6 to 20 ring atoms, alicyclic alkyl group substituted by straight-chain alkane and having 6 to 20 ring atoms, straight-chain alkane group substituted by aromatic ring and having 1 to 20 carbon atoms, or straight-chain alkane group substituted by alicyclic alkyl and having 1 to 20 carbon atoms, and at least one X₃ in formula (3) is not H.

In some embodiments, X₃ is independently selected at each occurrence from any one of H, aromatic ring group substituted by C1-C20 straight-chain alkane and having 6 to 10 ring atoms, alicyclic alkyl group substituted by C1-C20 straight-chain alkane and having 6 to 10 ring atoms, straight-chain alkane group substituted by aromatic ring that has 6 to 20 ring atoms and having 1 to 10 carbon atoms, and straight-chain alkane group substituted by alicyclic alkyl that has 6 to 20 ring atoms and having 1 to 20 carbon atoms, and at least one X₃ in formula (3) is not H.

In some embodiments, X₃ is independently selected at each occurrence from any one of H, aromatic ring group substituted by C1-C10 straight-chain alkane and having 6 to 10 ring atoms, alicyclic alkyl group substituted by C1-C10 straight-chain alkane and having 6 to 10 ring atoms, straight-chain alkane group substituted by aromatic ring that has 6 to 10 ring atoms and having 1 to 10 carbon atoms, and straight-chain alkane group substituted by alicyclic alkyl that has 6 to 10 ring atoms and having 1 to 10 carbon atoms, and at least one X₃ in formula (3) is not H.

Non-limiting examples of the alicyclic alkyl include cyclohexane and cyclooctane.

In some embodiments, the structure of formula (3) is shown as follows: 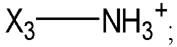 where X₃ is selected from any one of aromatic ring group substituted by straight-chain alkane and having 6 to 20 ring atoms or alicyclic alkyl group substituted by straight-chain alkane and having 6 to 20 ring atoms.

In some embodiments, X₃ is selected from any one of aromatic ring group substituted by C1-C20 straight-chain alkane and having 6 to 20 ring atoms or alicyclic alkyl group substituted by C1-C20 straight-chain alkane and having 6 to 20 ring atoms.

In some embodiments, X₃ is selected from any one of aromatic ring group substituted by C1-C10 straight-chain alkane and having 6 to 10 ring atoms or alicyclic alkyl group substituted by C1-C10 straight-chain alkane and having 6 to 10 ring atoms.

In some embodiments, the ionic compound has a structure represented by formulas (B) to (E): 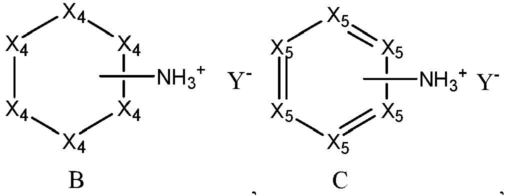 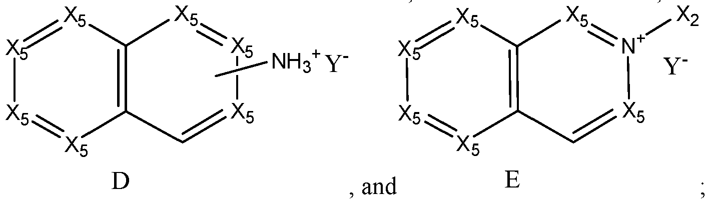
where X₄ is independently selected at each occurrence from CR₃R₄, and X₅ is independently selected at each occurrence from CR₅; and
R₃ to R₅ each are independently selected at each occurrence from any one of H or linear alkyl group having 1 to 30 carbon atoms; at least one R₃ or at least one R₄ in formula (A) is selected from linear alkyl group having 1 to 30 carbon atoms; and at least one R₅ in formula (C) and formula (D) is selected from linear alkyl group having 1 to 30 carbon atoms.

In some embodiments, R₃ to R₅ each are independently selected at each occurrence from any one of H or linear alkyl group having 1 to 20 carbon atoms; at least one R₃ or at least one R₄ in formula (A) is selected from linear alkyl group having 1 to 20 carbon atoms; and at least one R₅ in formula (C) and formula (D) is selected from linear alkyl group having 1 to 20 carbon atoms.

In some embodiments, R₃ to R₅ each are independently selected at each occurrence from any one of H or straight-chain alkyl group having 1 to 20 carbon atoms; at least one R₃ or at least one R₄ in formula (A) is selected from straight-chain alkyl group having 1 to 20 carbon atoms; and at least one R₅ in formula (D) and formula (E) is selected from straight-chain alkyl group having 1 to 20 carbon atoms.

R₃ to R₅ each are independently selected at each occurrence from any one of H or straight-chain alkyl group having 1 to 15 carbon atoms; at least R₃ or at least one R₄ in formula (B) is selected from straight-chain alkyl group having 1 to 15 carbon atoms; and at least one R₅ in formula (C) and formula (D) is selected from straight-chain alkyl group having 1 to 15 carbon atoms.

In some embodiments, R₃ to R₅ each are independently selected at each occurrence from any one of H or straight-chain alkyl group having 1 to 10 carbon atoms; at least one R₃ or at least one R₄ in formula (B) is selected from straight-chain alkyl group having 1 to 10 carbon atoms; and at least one R₅ in formula (C) and formula (D) is selected from straight-chain alkyl group having 1 to 10 carbon atoms.

In some embodiments, Y⁻ is selected from fluoroborate containing ion or fluorophosphate containing ion.

It should be noted that the symbol "⁻" in "Y⁻" denotes the formation of a negative valence state but does not limit the specific valence state value. "Y⁻" is not limited to a special valence state value in this application, provided that the algebraic sum of valence states of Y⁻ and cation parts of the above ionic compound is 0.

In a specific example, Y⁻ is selected from tetrafluoroborate ion or hexafluorophosphate ion.

The step of preparing the above ionic compound includes the following step S100.

Step S100. Make any one of compounds (T1) to (T3) react with a Y⁻ containing salt to obtain the above ionic compound: 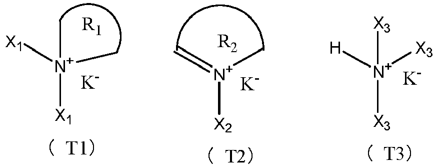 where K⁻ denotes a halogen ion, including but not limited to Br⁻, Cl⁻, and I⁻.

In some embodiments, the Y⁻ containing salt may be a Y⁻ containing metal salt, where a metal cation includes but is not limited to K⁻, Na⁻, and Li⁻.

In some embodiments, the reaction in step S100 is carried out in an alcohol solvent. The alcohol solvent includes but is not limited to one or more of methanol, ethanol, and propanol.

In some embodiments, the reaction in step S100 is carried out at 60°C for 12 h. Further, a method for preparing any one of the above compounds (T1) to (T3) is described by using (T2) as an example and specifically includes step S110:

Step S110. Make compound (T2-1) react with compound (T2-2) to obtain (T2): 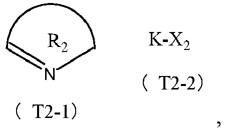 where R₂ and X₂ have the same meaning as the above, and K⁻ denotes a halogen group, including but not limited to Br, Cl, and I.

For a reaction mechanism of the reaction in step S110, refer to the Menshutkin reaction.

In some embodiments, the reaction in step S110 is carried out in an alcohol solvent. The alcohol solvent includes but is not limited to one or more of methanol, ethanol, and propanol.

(T1) and (T3) can also be prepared by using the foregoing synthesis thought. An embodiment of this application provides an application of the above ionic compounds as an ionic liquid.

The ionic compound of this application can be in a stable liquid form to form an ionic liquid, and can improve the stability of perovskite materials when used in the preparation of solar cells and thus improve the efficiency and stability of solar cells.

An embodiment of this application provides an application of the ionic compound represented by any one of formulas (3) to (5) in perovskite material preparation: 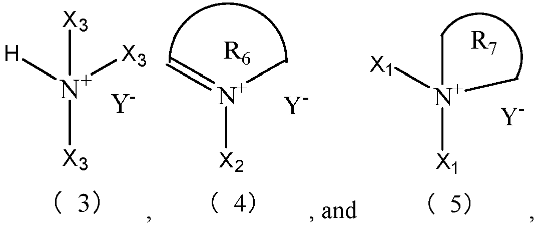
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

Formulas (1) and (3) have the same meaning as the above.

In some embodiments, in formula (4), X₂ is selected from linear alkane group having 1 to 25 carbon atoms, and R₆ and N are bonded to each other to form an aromatic heterocyclic ring unsubstituted or substituted by linear alkane, the aromatic heterocyclic ring having 6 to 25 ring atoms and containing only one N atom.

In some embodiments, in formula (4), X₂ is selected from straight-chain alkane group having 1 to 25 carbon atoms, and R₆ and N are bonded to each other to form an aromatic heterocyclic ring unsubstituted or substituted by linear alkane, the aromatic heterocyclic ring having 6 to 20 ring atoms and containing only one N atom.

In some embodiments, in formula (4), X₂ is selected from straight-chain alkane group having 1 to 10 carbon atoms, and R₆ and N are bonded to each other to form an aromatic heterocyclic ring unsubstituted or substituted by linear alkane, the aromatic heterocyclic ring having 6 to 15 ring atoms and containing only one N atom.

In some embodiments, R₇ and N are bonded to each other to form an alicyclic heterocyclic ring unsubstituted or substituted by linear alkane, the alicyclic heterocyclic ring having 6 to 20 ring atoms and containing only one N atom.

It should be noted that the above range is considered continuous and includes a minimum value and a maximum value of the range, as well as each value between such minimum value and maximum value, including but not limited to the point values in this embodiment. For example, the foregoing "6 to 20 ring atoms" may be 6, 7, 8, 9, 10, 11, 12, 13, 14, 15, 16, 17, 18, 18, or 20 ring atoms.

In some embodiments, R₇ and N are bonded to each other to form an alicyclic heterocyclic ring unsubstituted or substituted by C1-C20 linear alkane, the alicyclic heterocyclic ring having 6 to 10 ring atoms and containing only one N atom.

In some embodiments, R₇ and N are bonded to each other to form an alicyclic heterocyclic ring unsubstituted or substituted by C1-C10 straight-chain alkane, the alicyclic heterocyclic ring having 6 to 10 ring atoms and containing only one N atom. In some embodiments, the ionic compounds represented by formulas (3) to (5) have structures represented by formulas (A) to (F): 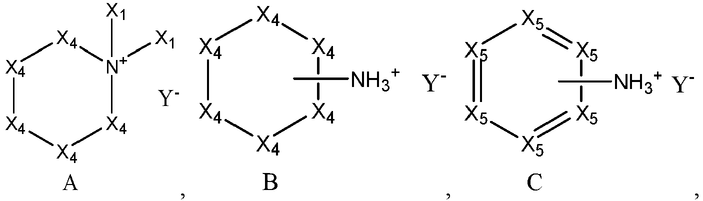 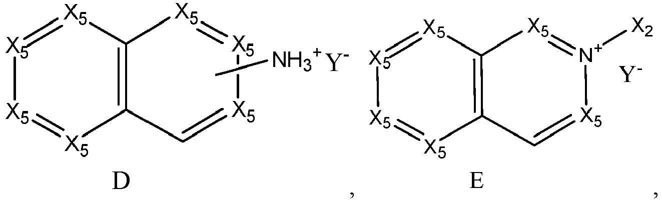 and 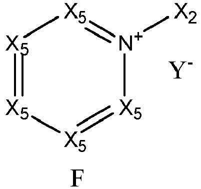 where X₄, X₅, and R₃ to R₅ have the same meaning as the above.

In some embodiments, the ionic compounds represented by formulas (3) to (5) each have a structure represented by any one of formulas (a) to (f):

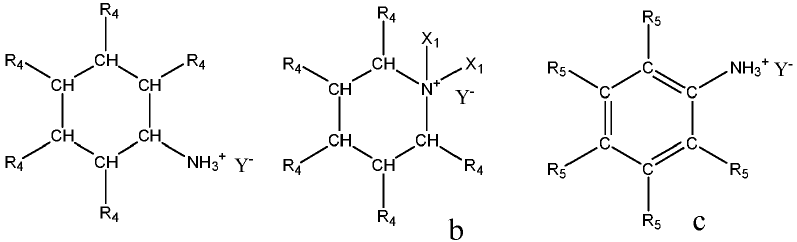

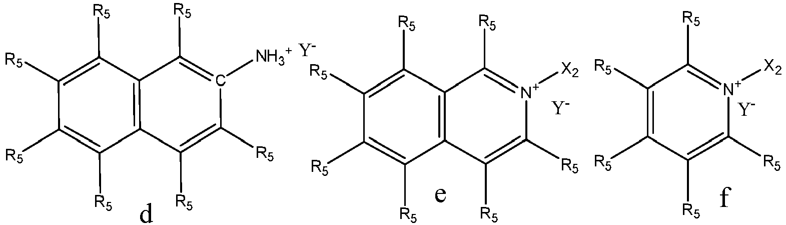

R₄ and R₅ have the same meaning as the above, at least one R₄ in formula (a) is selected from linear alkyl group having 1 to 30 carbon atoms; and at least one R₅ in formula (c) and formula (d) is selected from linear alkyl group having 1 to 30 carbon atoms.

An embodiment of the present invention further provides a perovskite precursor solution. Compositions of the perovskite precursor solution include a perovskite precursor and at least one of ionic compounds represented by formulas (3) to (5): 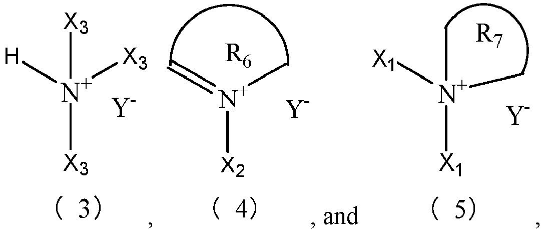
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

The ionic compounds represented by formulas (3) to (5) have the same structures as the above.

The above perovskite precursors may be perovskite precursors commonly used in the art for perovskite compound preparation. For example, the molecular formula of the perovskite compound is ABX₃, where A is a monovalent cation including one or more cations of lithium Li, sodium Na, potassium K, cesium Cs, rubidium Rb, amine group, and amidine group; B is a divalent cation including one or more cations of lead Pb, tin Sn, tungsten W, selenium Se, rhodium Rh, germanium Ge, arsenic As, indium In, and antimony Sb; and X is a monovalent anion including one or more anions of iodine I, bromine Br, and chloride Cl. Accordingly, a perovskite precursor of the perovskite compound has compositions A, B, and X whose ratio satisfies ABX₃, and specifically includes halide of A and halide of B. The halide of A includes one or more of iodide of A, bromide of A, and chloride of A. The halide of B includes one or more of iodide of B, bromide of B, and chloride of B, for example, at least one of lead iodide, formamidine iodide, cesium iodide, lead bromide, formamidine bromide, cesium bromide, lead chloride, formamidine chloride, and cesium chloride.

In some embodiments, the ratio of compositions of the above perovskite precursor satisfies the formula: FA₍₁₋ₓ₎CsₓPb(I_{1-y}Br_{y})₃, where 0<x<1 and 0≤y≤1.

In some embodiments, the above perovskite precursor solution further includes a solvent. The concentration of the above perovskite precursor solution is 0.8 mol/L to 1.5 mol/L.

Further, the solvent is an organic solvent and may be selected from one or more of DMF (N,N-dimethylacetamide), DMSO (dimethyl sulfoxide), NMP (1-methyl-2-pyrrolidone).

An embodiment of this application further provides a perovskite material. Compositions of the perovskite material include a perovskite compound and at least one of ionic compounds represented by formulas (3) to (5): 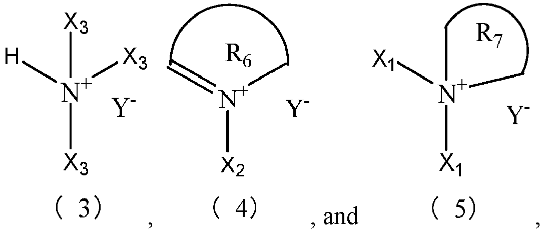
where X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

The above perovskite compound may be a perovskite compound commonly used in the art.

In some embodiments, the molecular formula of the perovskite compound is ABX₃, where A is a monovalent cation including one or more cations of lithium Li, sodium Na, potassium K, cesium Cs, rubidium Rb, amine group, and amidine group; B is a divalent cation including one or more cations of lead Pb, tin Sn, tungsten W, selenium Se, rhodium Rh, germanium Ge, arsenic As, indium In, and antimony Sb; and X is a monovalent anion including one or more anions of iodine I, bromine Br, and chloride Cl.

In some embodiments, the perovskite compound further includes element Pb, and a mass ratio of the ionic compound to the element lead in the perovskite compound is (1 to 10):100.

An embodiment of this application further provides a preparation method of the above perovskite material. The method includes the following steps S10 and S20:

Step S10. Mix a perovskite precursor, the ionic compound, and a solvent to obtain a perovskite precursor solution.

Step S20. Anneal the perovskite precursor solution to obtain the perovskite material.

In some embodiments, the concentration of the perovskite precursor solution is 0.8 mol/L to 1.5 mol/L.

In some embodiments, the solvent is an organic solvent and may be selected from one or more of DMF (N,N-dimethylacetamide), DMSO (dimethyl sulfoxide), NMP (1-methyl-2-pyrrolidone).

In some embodiments, the temperature for the annealing treatment is 70°C to 200°C and the time thereof is 3 min to 60 min.

In some embodiments, the perovskite material is in a film form. Before the annealing treatment, step S20 further includes a step of applying the perovskite precursor solution on a substrate.

In some embodiments, the coating step may be performed through a spin coating, scrape coating, slot die coating, or another method.

An embodiment of the present invention provides a solar cell. The solar cell includes the foregoing perovskite material.

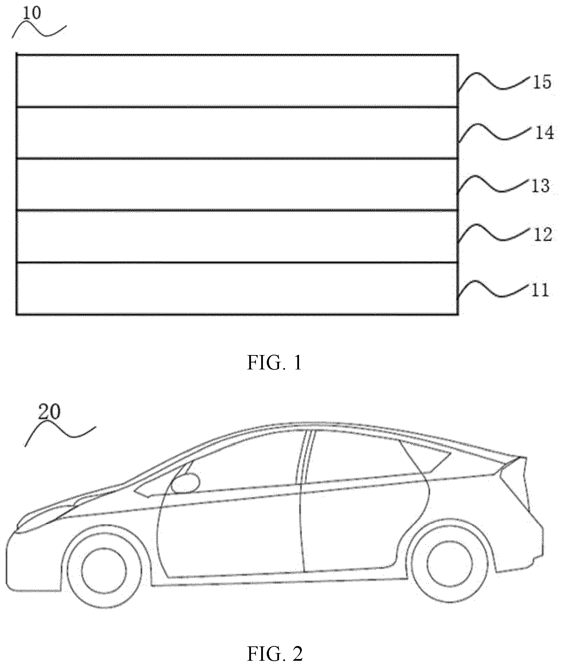

Refer to FIG. 1. FIG. 1 is a schematic diagram of a solar cell 10 according to an embodiment of this application. The solar cell 10 includes a first electrode 11, an electron transport layer 12, a perovskite layer 13, a hole transport layer 14, and a second electrode 15 that are stacked in sequence. The composition of the perovskite layer 13 includes the foregoing perovskite material.

In some embodiments, the thickness of the perovskite layer 13 is 300 nm to 600 nm.

In some embodiments, the first electrode 11 is a transparent conductive electrode. Further, the first electrode 11 may be made of FTO, ITO, AZO, BZO, IZO, or a composite conductive film. The composite conductive film includes a transparent polymer substrate and a transparent inorganic conductive film that are stacked. The transparent inorganic conductive film may be made of FTO, ITO, AZO, BZO, or IZO, and the transparent polymer substrate material may be made of PET (polyethylene terephthalate), PI (polyimide), or the like.

The composition of the electron transport layer 12 may be electron transport materials commonly used in the art, and includes but is not limited to one or more of TiO₂, SnO₂, ZnO, and [6,6]-phenyl-C61-butyric acid iso-methyl ester (PCBM).

The composition of the hole transport layer 14 may be a hole transport material commonly used in the art, and includes but is not limited to one or more of 2,2',7,7'-tetrakis [N,N-bi(4-methoxyphenyl)amino]-9,9'-spiro-difluorene (Spiro-OMeTAD), poly(triarylamine) (PTAA), NiOx, poly(3,4-ethylenedioxythiophene):polystyrene sulfonate (PEDOT:PSS), and WO₃.

In some embodiments, the composition of the second electrode 15 includes one or more of Au, Ag, Cu, Al, Ni, Cr, Bi, Pt, Mg, MoOs, ITO, FTO, and AZO.

Specifically, a step of preparing the solar cell 10 includes forming the electron transport layer 12, the perovskite layer 13, the hole transport layer 14, and 15 the second electrode on the surface of the first electrode 11 in sequence.

An embodiment of the present invention further provides an electric apparatus, and the electric apparatus includes the foregoing solar cell.

The solar cell has high photoconversion efficiency and good stability, and therefore, can improve the efficiency and life of the electric apparatus.

In some embodiments, the solar cell may be used as a power source of the electric apparatus or an energy storage unit of the electric apparatus.

Further, the electric apparatus may include a mobile device such as a mobile phone, a notebook computer, an electric vehicle, an electric train, a ship, a satellite system, an energy storage system, but is not limited thereto.

FIG. 2 shows an electric apparatus as an example. This electric apparatus 20 is a battery electric vehicle, a hybrid electric vehicle, a plug-in hybrid electric vehicle, or the like.

In another example, the electric apparatus may be a mobile phone, a tablet computer, a notebook computer, or the like.

The following provides specific examples.

### Example 1

### (1) The following compound (1): N-octyl-4-methylquinoline tetrafluoroborate was provided.

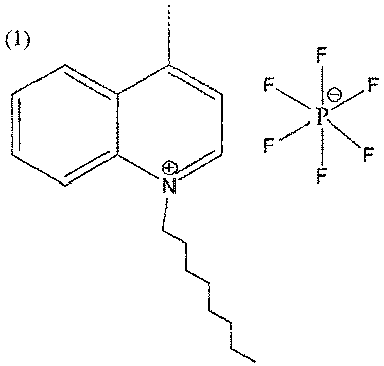

The specific preparation procedure was as follows:
5.73 g (1 mol) 4-methylquinoline (CAS: 491-35-0), 8.5 g (1.1 mol) bromooctane (CAS: 111-83-1), and 40 mL methanol were added into a 250 mL three-necked round bottom flask for reaction at 70°C for 6 h. A resulting reaction mixture was poured into a 250 mL rotary evaporator and distilled under reduced pressure for 1 h to obtain a crude product. The crude product was then dried in a vacuum drying oven at 110°C for 12 h to obtain an intermediate N-octyl-4-methylquinoline bromide.
18.4 g (1 mol) KPF₆ (CAS: 17084-13-8), 26.9 g (0.8 mol) intermediate N-octyl-4-methylquinoline bromide, and 100 mL methanol were added to a 250 mL three-necked flask and stirred for reaction for 12 h at 60°C. The resulting mixture was transferred to a separatory funnel, and rinsed with methanol for five times. Then anhydrous magnesium sulfate was added to remove water, suction filtration was performed to collect filtrate, and the methanol was removed by using the rotary evaporator. The resulting product was dried in the vacuum drying oven at 110°C for 12 h to obtain compound (1).

1H nuclear magnetic resonance test was performed on compound (1) and 1H nuclear magnetic resonance data was as follows:
1H NMR: δ 0.87 (3H, t, J = 7.0 Hz), 1.17-1.39 (10H, 1.23 (tt, J = 6.9, 6.5 Hz), 1.24 (tt, J = 7.0, 6.9 Hz), 1.25 (tt, J = 7.0, 6.5 Hz), 1.28 (h, J = 7.0 Hz), 1.32 (tt, J = 7.0, 6.8 Hz)), 1.90 (2H, tt, J = 7.6, 6.8 Hz), 2.58 (3H, s), 4.54 (2H, t, J = 7.6 Hz), 7.48-7.64 (2H, 7.54 (td, J = 8.5, 1.7 Hz), 7.57 (ddd, J = 8.5, 3.9, 1.5 Hz)), 7.74 (1H, dd, J = 5.4, 0.4 Hz), 7.98 (1H, ddt, J = 3.9, 1.7, 0.5 Hz), 8.91-9.10 (2H, 8.97 (dd, J = 5.4, 0.5 Hz), 9.04 (ddt, J = 8.4, 1.5, 0.4 Hz)).

### (2) Specific steps for solar cell preparation were as follows:

1. 2 cm × 2 cm FTO conductive glass was taken and subjected to laser etching, with an insulating area reserved, and then subjected to ultrasound in turn with deionized water, detergent, ethanol, isopropanol, acetone, ethanol, and deionized water for 20 min. The resulting product was dried with N₂ for later use.
2. A clean FTO substrate was spin-coated with a SnO₂ precursor solution at a speed of 3000 rpm. The resulting product was annealed at 150°C for 15 min to form a SnO₂ substrate, and the substrate was subjected to natural cooling for later use.
3. Compound (1) was added to FA_{0.85}Cs_{0.15}Pb(I_{0.9}Br_{0.1})₃ perovskite precursor solution with a concentration of 1.25 M, so as to form a perovskite precursor solution which was sealed for later use, where a molar ratio of compound (1) to Pb was 1%.
4. The surface of the SnO₂ substrate subjected to 15 min ultraviolet ozone treatment was blown clean, and spin-coated with the foregoing perovskite precursor solution containing an ionic liquid at a rotary speed of 4000 rpm for 20s. The resulting product was subjected to vacuumization for 30s, annealed on a 120°C hot table for 45 min, and naturally cooled to obtain a 500-nm-thick perovskite light-absorbing layer.
5. The perovskite light-absorbing layer was spin-coated with a spiro-OMeTAD solution at a rotary speed of 3000 rpm to form a 200-nm-thick hole transport layer.
6. An Ag electrode was deposited on the hole transport layer with a patterned mask plate by using the thermal evaporation method, to obtain a perovskite solar cell denoted as A1.

### (3) Performance test was performed on the solar cell prepared, and the specific steps were as follows:

Under standard simulated solar radiation (AM1.5G, 100 mW/cm²), the photoelectric conversion efficiency of the solar cell prepared (that is, the standing time was 0 h) was tested. The test results are shown in Table 1. Further, the solar cell was placed in an environment with a room temperature of 25°C and humidity of 85% for 200 h, and then subjected to J-V test again to check its stability. See Table 1 for the specific results.

In Table 1, VOC is open circuit voltage, JSC is short circuit current, FF is fill factor, and PCE is power conversion efficiency.

### Example 2

Example 2 was the same as Example 1 except that compound (1) in Example 1 was replaced with equimolar compound (2) in Example 2. The structure of compound (2) is shown as follows:

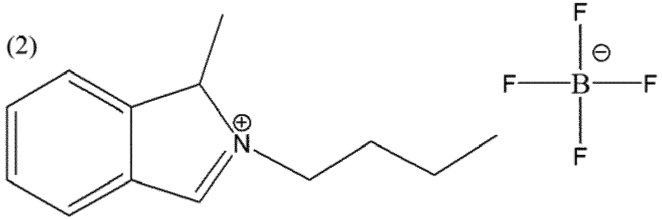

Compound (2) was prepared in the same way as compound (1) in Example 1 except that 4-methylquinoline was replaced with equimolar 1-methylisoindole (CAS: 72590-71-7), bromooctane was replaced with equimolar bromobutane (CAS: 105-65-9), and KPF₆ was replaced with equimolar NaBF₄.

1H nuclear magnetic resonance test was performed on compound (2) obtained and 1H nuclear magnetic resonance data was as follows:
1H NMR: δ 0.89 (3H, t, J = 7.1 Hz), 1.39 (2H, qt, J = 7.1, 7.0 Hz), 1.83 (2H, tt, J = 7.5, 7.0 Hz), 2.58 (3H, s), 3.60 (2H, t, J = 7.5 Hz), 4.96 (2H, d, J = 14.4 Hz), 7.34 (1H, dd, J = 8.4, 1.5 Hz), 7.77 (1H, t, J = 8.4 Hz), 8.74-8.93 (2H, 8.79 (s), 8.87 (dd, J = 8.4, 1.5 Hz)).

A solar cell prepared was denoted as A2. See Table 1 for specific results.

### Example 3

Example 3 was the same as Example 1 except that compound (1) in Example 1 was replaced with equimolar compound (3) in Example 3. The structure of compound (3) is shown as follows:

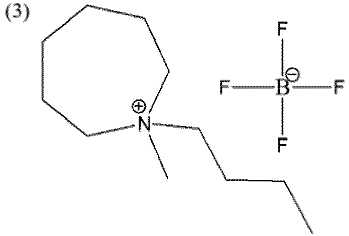

Compound (3) was prepared in the same way as compound (1) in Example 1 except that 4-methylquinoline was replaced with equimolar N-butylhexamethyleneimine (CAS: 15753-35-2), bromooctane was replaced with equimolar bromomethane (CAS: 74-83-9), and KPF₆ was replaced with equimolar NaBF₄.

1H nuclear magnetic resonance test was performed on a target product compound (3) obtained and 1H nuclear magnetic resonance data was as follows:
1H NMR: δ 0.89 (3H, t, J = 6.5 Hz), 1.35 (2H, tq, J = 6.6, 6.5 Hz), 1.43-1.65 (4H, 1.51 (ddddd, J = 15.2, 4.7, 4.5, 2.3, 1.6 Hz), 1.55 (ddddd, J = 15.2, 9.7, 8.8, 2.3, 1.6 Hz)), 1.81-2.07 (6H, 1.88 (tt, J = 7.7, 6.6 Hz), 1.94 (ddddd, J = 13.5, 8.8, 6.8, 5.4, 2.3 Hz), 1.98 (ddddd, J = 13.5, 5.2, 4.7, 2.3, 1.5 Hz)), 2.97 (3H, s), 3.10 (2H, t, J = 7.7 Hz), 3.18-3.39 (4H, 3.26 (ddd, J = 13.2, 5.4, 5.2 Hz), 3.31 (ddd, J = 13.2, 6.8, 1.5 Hz)).

A solar cell prepared was denoted as A3. See Table 1 for specific results.

### Example 4

Example 4 was the same as Example 1 except that compound (1) in Example 1 was replaced with equimolar compound (4) (CAS: 343952-33-0) in Example 4, having a structure as follows:

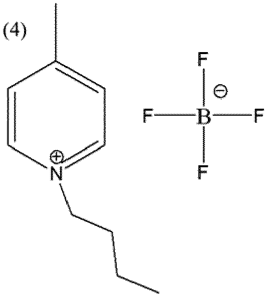

A solar cell prepared was denoted as A4. See Table 1 for specific results.

### Example 5

Example 5 was the same as Example 1 except that compound (1) in Example 1 was replaced with equimolar compound (5) (CAS: 886439-34-5) in Example 5. The structure of compound (5) is shown as follows:

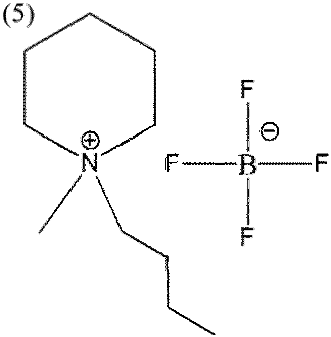

A solar cell prepared was denoted as A5. See Table 1 for specific results.

### Example 6

Example 6 was the same as Example 1 except that compound (1) in Example 1 was replaced with equimolar compound (6) in Example 6. The structure of compound (6) is shown as follows:

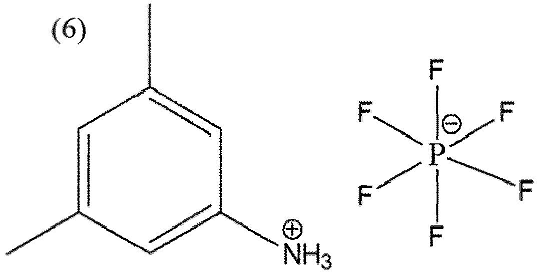

Compound (6) was prepared in the same way as compound (1) in Example 1 except that 4-methylquinoline was replaced with equimolar 3,5-dimethylaniline (CAS: 108-69-0) and bromooctane was replaced with equimolar hydrochloric acid (CAS: 7647-01-0).

1H nuclear magnetic resonance test was performed on a target product compound (6) obtained and 1H nuclear magnetic resonance data was as follows:
1H NMR: δ 2.27 (6H, s), 6.47 (2H, dd, J = 2.7, 2.3 Hz), 6.73 (1H, t, J = 2.7 Hz).

It should be noted that free hydrogen of the amino group in compound (6) was not displayed in nuclear magnetic resonance.

A solar cell prepared was denoted as A6. See Table 1 for specific results.

### Example 7

Example 7 was the same as Example 1 except that compound (1) in Example 1 was replaced with equimolar compound (7) in Example 7. The structure of compound (7) is shown as follows:

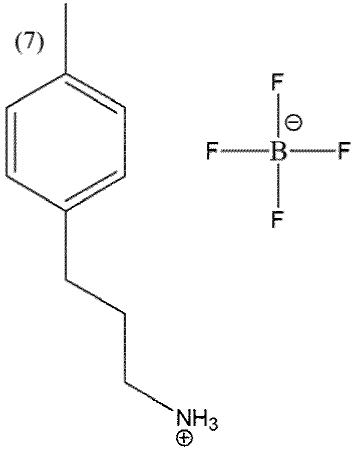

Compound (7) was prepared in the same way as compound (1) in Example 1 except that 4-methylquinoline was replaced with equimolar 4-methylamphetamine (CAS: 108-69-0), bromooctane was replaced with equimolar hydrochloric acid (CAS: 7647-01-0), and KPF₆ was replaced with equimolar NaBF₄.

1H nuclear magnetic resonance test was performed on a target product compound (7) obtained and data was as follows:
1H NMR: δ 1.63 (2H, tt, J = 7.5, 7.3 Hz), 2.24 (3H, s), 2.51-2.70 (4H, 2.57 (t, J = 7.5 Hz), 2.64 (t, J = 7.3 Hz)), 7.04 (2H, ddd, J = 8.0, 1.4, 0.5 Hz), 7.19 (2H, ddd, J = 8.0, 1.3, 0.5 Hz).

It should be noted that free hydrogen of the amino group in compound (7) was not displayed in nuclear magnetic resonance.

A solar cell prepared was denoted as A7. See Table 1 for specific results.

### Example 8

Example 8 was the same as Example 1 except that compound (1) in Example 1 was replaced with equimolar compound (8) in Example 8. The structure of compound (8) is shown as follows:

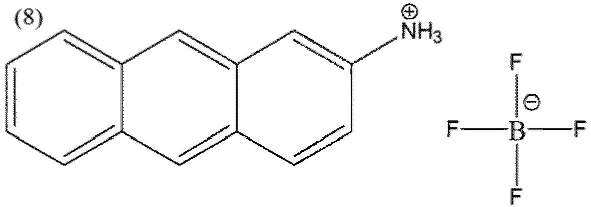

Compound (8) was prepared in the same way as compound (1) in Example 1 except that 4-methylquinoline was replaced with equimolar 2-aminoanthracene (CAS: 613-13-8), bromooctane was replaced with equimolar hydrochloric acid (CAS: 7647-01-0), and KPF₆ was replaced with equimolar NaBF₄.

1H nuclear magnetic resonance test was performed on a target product obtained and data was as follows:
1H NMR: δ 6.87 (1H, ddd, J = 8.9, 1.9, 0.5 Hz), 7.37-7.58 (3H, 7.44 (dddd, J = 8.0, 7.5, 1.2, 0.5 Hz), 7.47 (dddd, J = 7.9, 7.5, 1.2, 0.5 Hz), 7.51 (ddt, J = 8.9, 1.6, 0.4 Hz)), 7.73-7.90 (3H, 7.79 (dddt, J = 7.9, 2.6, 1.2, 0.5 Hz), 7.83 (dddt, J = 8.0, 1.5, 1.2, 0.5 Hz), 7.84 (tt, J = 1.9, 0.4 Hz)), 8.17 (1H, ddquint, J = 2.6, 1.9, 0.5 Hz), 8.37 (1H, ddh, J = 1.6, 1.5, 0.5 Hz).

It should be noted that free hydrogen of the amino group in compound (8) was not displayed in nuclear magnetic resonance.

A solar cell prepared was denoted as A8. See Table 1 for specific results.

### Example 9

Example 9 was the same as Example 1 except that compound (1) in Example 1 was replaced with equimolar compound (9) in Example 9. The structure of compound (9) is shown as follows:

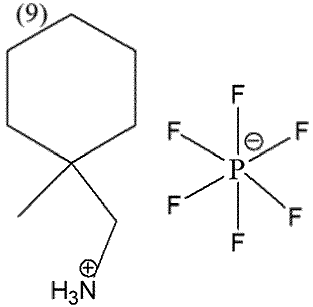

Compound (8) was prepared in the same way as compound (1) in Example 1 except that 4-methylquinoline was replaced with equimolar (1-methylcyclohexyl)methylamine (CAS: 3913-98-2) and bromooctane was replaced with equimolar hydrochloric acid(CAS: 7647-01-0).

1H nuclear magnetic resonance test was performed on a target product obtained and data was as follows:
1H NMR: δ 1.01 (3H, s), 1.15-1.44 (10H, 1.23 (dt, J = 12.8, 6.5 Hz), 1.29 (dtt, J = 11.8, 10.3, 2.8 Hz), 1.31 (dquint, J = 11.8, 2.8 Hz), 1.37 (dtt, J = 11.3, 6.5, 2.8 Hz), 1.37 (dtt, J = 11.3, 6.5, 2.8 Hz), 1.36 (dt, J = 12.8, 2.8 Hz)), 2.60 (2H, s).

It should be noted that free hydrogen of the amino group in compound (9) was not displayed in nuclear magnetic resonance.

A solar cell prepared was denoted as A9. See Table 1 for specific results.

### Comparative Example 1

Comparative Example 1 was the same as Example 1 except that compound (1) in Example 1 was replaced with the following equimolar compound in Comparative Example 1, and a molar ratio of the compound to Pb in the perovskite precursor solution was 3%.

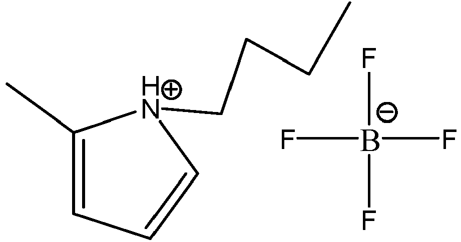

The compound was prepared in the same way as compound (1) in Example 1 except that 4-methylquinoline was replaced with equimolar 2-methylpyrrole (CAS: 636-41-9), bromooctane was replaced with equimolar bromobutane (CAS: 105-65-9), and KPF₆ was replaced with equimolar NaBF₄.

1H nuclear magnetic resonance test was performed on a target product obtained and data was as follows:
1H NMR: δ 0.88 (3H, t, J = 6.6 Hz), 1.33 (2H, h, J = 6.6 Hz), 1.71 (2H, tt, J = 7.4, 6.6 Hz), 2.18 (3H, s), 3.66 (2H, t, J = 7.4 Hz), 5.93-6.07 (2H, 5.98 (dd, J = 3.2, 1.6 Hz), 6.01 (dd, J = 3.2, 3.0 Hz)), 6.98 (1H, dd, J = 3.0, 1.6 Hz).

A solar cell prepared was denoted as B1. See Table 1 for specific results.

### Comparative Example 2

Comparative Example 2 was the same as Comparative Example 1 except that a molar ratio of the following compound to Pb in the perovskite precursor solution of Comparative Example 2 was 1%.

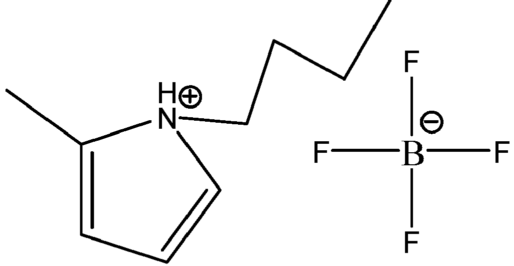

A solar cell prepared was denoted as B2. See Table 1 for specific results.

### Comparative Example 3

Comparative Example 3 was the same as Example 1 except that compound (1) in Example 1 was replaced with the following equimolar compound in Comparative Example 3.

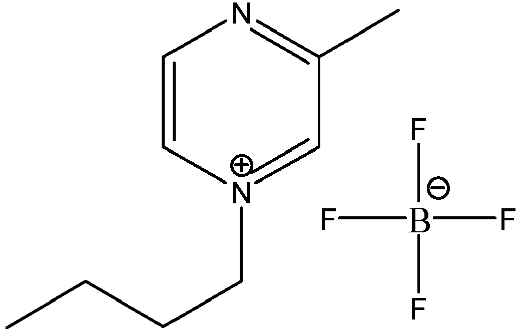

The compound was prepared in the same way as compound (1) in Example 1 except that 4-methylquinoline was replaced with equimolar 2-methylpyrazine (CAS: 109-08-0), bromooctane was replaced with equimolar bromobutane (CAS: 105-65-9), and KPF₆ was replaced with equimolar NaBF₄.

1H nuclear magnetic resonance test was performed on a target product obtained and data was as follows:
1H NMR: δ 0.89 (3H, t, J = 7.1 Hz), 1.35 (2H, qt, J = 7.1, 7.0 Hz), 1.85 (2H, tt, J = 7.0, 6.8 Hz), 2.75 (3H, s), 4.35 (2H, t, J = 6.8 Hz), 8.41-8.67 (3H, 8.47 (dd, J = 6.9, 1.8 Hz), 8.54 (dd, J = 1.8, 0.5 Hz), 8.61 (dd, J = 6.9, 0.5 Hz)). A solar cell prepared was denoted as B3. See Table 1 for specific results.

### Comparative Example 4

Comparative Example 4 was the same as Example 1 except that compound (1) in Example 1 was replaced with the following equimolar compound in Comparative Example 4.

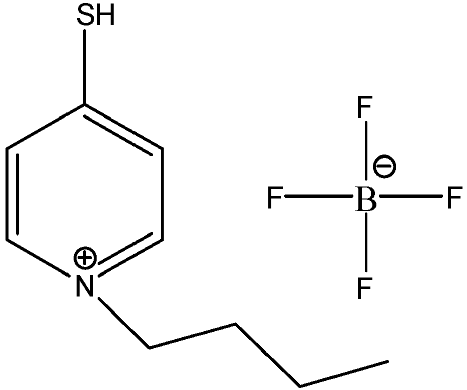

The compound was prepared in the same way as compound (1) in Example 1 except that 4-methylquinoline was replaced with equimolar 4-mercaptopyridine (CAS: 4556-23-4), bromooctane was replaced with equimolar bromobutane (CAS: 105-65-9), and KPF₆ was replaced with equimolar NaBF₄.

1H nuclear magnetic resonance test was performed on a target product obtained and data was as follows:
1H NMR: δ 0.89 (3H, t, J = 7.1 Hz), 1.35 (2H, qt, J = 7.1, 7.0 Hz), 1.80 (2H, tt, J = 7.4, 7.0 Hz), 4.37 (2H, t, J = 7.4 Hz), 7.79 (2H, ddd, J = 6.9, 1.9, 0.5 Hz), 8.66 (2H, ddd, J = 6.9, 1.7, 0.5 Hz). A solar cell prepared was denoted as B4. See Table 1 for specific results.

### Comparative Example 5

Comparative Example 5 was the substantially same as Example 1 in steps and conditions except that in Comparative Example 5, the ionic compound (1) was not added to the perovskite precursor solution.

A solar cell prepared was denoted as B5. See Table 1 for specific results.

The test results of examples and comparative examples are shown in Table 1 below.

**Table 1**

| | Left standing for 0 h | | | | Left standing for 200 hours | | | |
|---|---|---|---|---|---|---|---|---|
| | Voc (V) | Jsc (mA/cm²) | FF (%) | PCE (%) | Voc (V) | Jsc (mA/cm²) | FF (%) | PCE (%) |
| Example 1 | 1.05 | 22.36 | 75.13 | 17.63 | 1.05 | 21.95 | 74.93 | 17.26 |
| Example 2 | 1.04 | 22.93 | 76.23 | 18.17 | 1.02 | 21.45 | 75.32 | 16.47 |
| Example 3 | 1.04 | 21.55 | 75.21 | 16.85 | 1.03 | 21.03 | 74.5 | 16.13 |
| Example 4 | 1.09 | 23.15 | 74.06 | 18.68 | 1.07 | 22.88 | 73.58 | 18.01 |
| Example 5 | 1.06 | 22.73 | 75.53 | 18.19 | 1.05 | 22.31 | 75.01 | 17.57 |
| Example 6 | 1.07 | 22.89 | 74.82 | 18.32 | 1.05 | 21.96 | 73.71 | 16.99 |
| Example 7 | 1.08 | 22.68 | 75.01 | 18.37 | 1.07 | 21.43 | 73.22 | 16.78 |
| Example 8 | 1.08 | 22.31 | 74.96 | 18.05 | 1.06 | 21.08 | 72.21 | 16.13 |
| Example 9 | 1.05 | 22.14 | 74.36 | 17.28 | 1.03 | 21.65 | 73.11 | 16.30 |
| Comparative Example 1 | 0.99 | 20.91 | 72.65 | 15.03 | 0.98 | 20.65 | 72.09 | 14.59 |
| Comparative Example 2 | 1.01 | 21.44 | 73.19 | 15.84 | 0.99 | 20.88 | 72.50 | 14.98 |
| Comparative Example 3 | 0.98 | 20.83 | 71.22 | 14.53 | 0.97 | 20.46 | 70.42 | 13.97 |
| Comparative Example 4 | 0.99 | 20.50 | 70.68 | 14.34 | 0.98 | 20.01 | 70.21 | 13.77 |
| Comparative Example 5 | 0.98 | 20.08 | 70.35 | 13.84 | 0.97 | 19.38 | 69.45 | 13.06 |

It can be seen from the results in Table 1 that when the ionic compound of this application is used for perovskite layer preparation, the stability of the perovskite layer can be improved and defects can be reduced, and thus the efficiency and stability of the solar cell can be improved.

In conclusion, it should be noted that the foregoing embodiments are for description of the technical solutions of this application only rather than for limiting this application. Although this application has been described in detail with reference to the foregoing embodiments, persons of ordinary skill in the art should appreciate that they can still make modifications to the technical solutions described in the embodiments or make equivalent replacements to some or all technical features thereof without departing from the scope of the technical solutions of the embodiments of this application. All such modifications and equivalent replacements shall fall within the scope of claims and specification of this application. In particular, as long as there is no structural conflict, the various technical features mentioned in the embodiments can be combined in any manner. This application is not limited to the specific embodiments disclosed in this specification but includes all technical solutions falling within the scope of the claims.

## Claims

1. An ionic compound, **characterized in that** the ionic compound has a structure represented by any one of formulas (1) to (3): 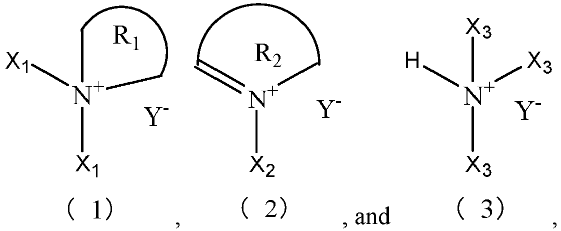
wherein X₁ is independently selected at each occurrence from any one of H or linear alkane group having 1 to 50 carbon atoms; R₁ and N are bonded to each other to form an alicyclic heterocyclic ring unsubstituted or substituted by linear alkane, the alicyclic heterocyclic ring having 7 to 50 ring atoms and containing only one N atom; and at least one X₁ in formula (1) is not H;
X₂ is selected from linear alkane group having 1 to 50 carbon atoms, and R₂ and N are bonded to each other to form an aromatic heterocyclic ring unsubstituted or substituted by linear alkane, the aromatic heterocyclic ring having 7 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

2. The ionic compound according to claim 1, **characterized in that** R₁ and N are bonded to each other to form an alicyclic heterocyclic ring unsubstituted or substituted by linear alkane, the alicyclic heterocyclic ring having 7 to 20 ring atoms and containing only one N atom; and/or
R₂ and N are bonded to each other to form a dense aromatic heterocyclic ring unsubstituted or substituted by linear alkane, the dense aromatic heterocyclic ring having 8 to 20 ring atoms and containing only one N atom.

3. The ionic compound according to claim 1, **characterized in that** X₃ is independently selected at each occurrence from any one of H, aromatic ring group substituted by straight-chain alkane and having 6 to 20 ring atoms, alicyclic alkyl group substituted by straight-chain alkane and having 6 to 20 ring atoms, straight-chain alkane group substituted by aromatic ring and having 1 to 20 carbon atoms, or straight-chain alkane group substituted by alicyclic alkyl and having 1 to 20 carbon atoms, and at least one X₃ in formula (3) is not H.

4. The ionic compound according to any one of claims 1 to 3, **characterized in that** the structure of formula (3) is shown as follows: 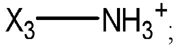 wherein X₃ is selected from any one of aromatic ring group substituted by straight-chain alkane and having 6 to 20 ring atoms or alicyclic alkyl group substituted by straight-chain alkane and having 6 to 20 ring atoms.

5. The ionic compound according to any one of claims 1 to 4, **characterized in that** the ionic compound has a structure represented by formulas (B) to (E): 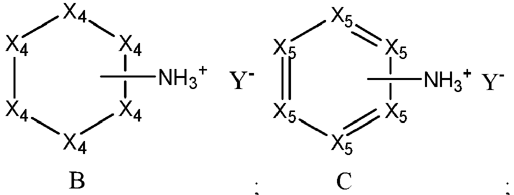 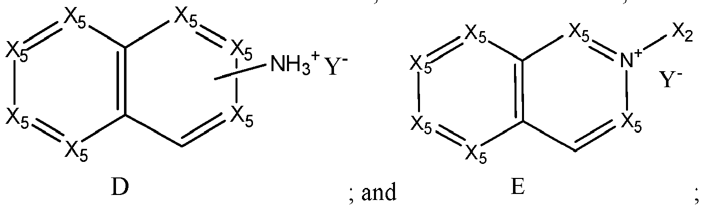
wherein X₄ is independently selected at each occurrence from CR₃R₄, and X₅ is independently selected at each occurrence from CR₅; and
R₃ to R₅ each are independently selected at each occurrence from any one of H or linear alkyl group having 1 to 30 carbon atoms; at least one R₃ or at least one R₄ in formula (B) is selected from linear alkyl group having 1 to 30 carbon atoms; and at least one R₅ in formula (C) and formula (D) is selected from linear alkyl group having 1 to 30 carbon atoms.

6. The ionic compound according to claim 5, **characterized in that** R₃ to R₅ each are independently selected at each occurrence from H or straight-chain alkyl group having 1 to 15 carbon atoms; at least R₃ or at least one R₄ in formula (B) is selected from straight-chain alkyl group having 1 to 15 carbon atoms; and at least one R₅ in formula (C) and formula (E) is selected from straight-chain alkyl group having 1 to 15 carbon atoms.

7. The ionic compound according to any one of claims 1 to 6, **characterized in that** Y⁻ is selected from fluoroborate containing ion or fluorophosphate containing ion.

8. An application of the ionic compound according to any one of claims 1 to 7 in an ionic liquid.

9. An application of the ionic compound represented by any one of formulas (3) to (5) in perovskite material preparation: 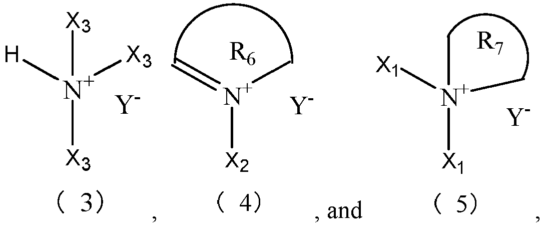
wherein X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (5) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

10. A perovskite precursor solution, **characterized in that** compositions of the perovskite precursor solution comprise a perovskite precursor and at least one of ionic compounds represented by formulas (3) to (5): 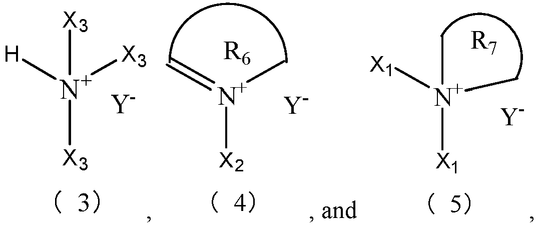
wherein X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

11. A perovskite material, **characterized in that** compositions of the perovskite material comprise a perovskite compound and at least one of ionic compounds represented by formulas (3) to (5): 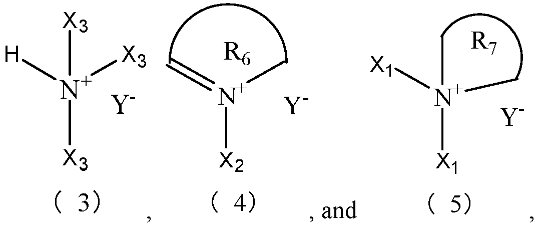
wherein X₁ is independently selected at each occurrence from any one of H or linear alkane group having 1 to 50 carbon atoms; R₇ and N are bonded to each other to form an alicyclic heterocyclic ring unsubstituted or substituted by linear alkane, the alicyclic heterocyclic ring having 6 to 50 ring atoms and containing only one N atom; and at least one X₁ in formula (1) is not H;
X₂ is selected from linear alkane group having 1 to 50 carbon atoms, and R₆ and N are bonded to each other to form an aromatic heterocyclic ring unsubstituted or substituted by linear alkane, the aromatic heterocyclic ring having 6 to 50 ring atoms and containing only one N atom;
X₃ is independently selected at each occurrence from any one of H, aromatic ring group substituted by linear alkane and having 6 to 50 ring atoms, alicyclic alkyl group substituted by linear alkane and having 6 to 50 ring atoms, linear alkane group substituted by aromatic ring and having 1 to 50 carbon atoms, or linear alkane group substituted by alicyclic alkyl and having 1 to 50 carbon atoms, and at least one X₃ in formula (3) is not H; and
Y⁻ is a fluorine-containing anion.

12. The perovskite material according to claim 11, **characterized in that** a mass ratio of the ionic compound to element lead in the perovskite compound is (1 to 10):100.

13. A preparation method for the perovskite material according to claim 11 or 12, **characterized by** comprising the following steps:
mixing a perovskite precursor, the ionic compound, and a solvent to obtain a perovskite precursor solution; and
annealing the perovskite precursor solution to obtain the perovskite material.

14. A solar cell, **characterized in that** the solar cell comprises the perovskite material according to either claim 11 or 12.

15. An electric apparatus, **characterized in that** the electric apparatus comprises the solar cell according to claim 14.
